# EUROPEAN PATENT SPECIFICATION

(11) **EP 3 097 907 B1**
(45) Date of publication and mention of the grant of the patent: **15.08.2018**
(21) Application number: 16020194.3
(22) Date of filing: 27.05.2016
(51) Int. Cl.: A61K 9/00, A61K 47/00, A61K 47/10, A61K 9/06, A61K 31/167, A61K 31/192

(54) **A GEL FORMULATION COMPRISING ANALGESIC AND ANESTHESIC AGENTS**
GELFORMULIERUNG MIT ANALGETISCHEN UND ANÄSTHETISCHEN WIRKSTOFFEN
FORMULATION DE GEL COMPRENANT DES AGENTS ANALGÉSIQUES ET ANESTHÉSIANTS

(30) Priority: 28.05.2015 TR 201506479
(43) Date of publication of application: 30.11.2016
(73) Proprietor: Abdi Ibrahim Ilac Sanayi ve Ticaret A.S., 34500 Esenyurt/Istanbul (TR)
(72) Inventor: Farsi, Ferhad, 34500 Esenyurt/Istanbul (TR); Dogru, Bugra, 34500 Esenyurt/Istanbul (TR); Kumar, Udaya Dude, 34500 Esenyurt/Istanbul (TR); Almareddy, Srinivasa Reddy, 34500 Esenyurt/Istanbul (TR); Akdag, Kadriye, 34500 Esenyurt/Istanbul (TR); Evin, Handan, 34500 Esenyurt/Istanbul (TR)

(56) References cited:
- EP-A2- 1 405 646
- US-A1- 2009 048 296
- MARRA D E ET AL: "Systemic toxicity from topically applied Lidocaine in conjunction with fractional photothermolysis", ARCHIVES OF DERMATOLOGY, AMERICAN MEDICAL ASSOCIATION, US, vol. 142, 1 January 2006 (2006-01-01), pages 1024-1026, XP002509848, ISSN: 0003-987X, DOI: 10.1001/ARCHDERM.142.8.1024

## Description

The invention relates to the formulations in gel form comprising an analgesic and an anesthetic agents, and the topical application of these formulations. More specifically, the invention relates to gel formulations comprising naproxen and lidocaine.

### Technical Field of Invention

Naproxen is an analgesic, ant-inflammatory, antipyretic drug that is a member of non-steroidal anti-inflammatory drugs (NSAIDs) group.

It was first described in the patent document numbered US3904682. Chemical name is (+)-2-(6-Methoxy-2-naphthyl) propionic acid and its chemical structure is as shown in formula I.

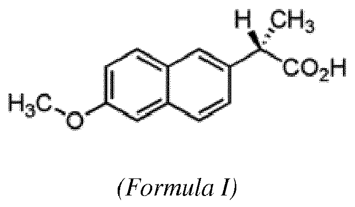

Naproxen is white or off-white, almost odorless, crystalline powder. Practically insoluble in water, soluble in alcohol, chloroform, and methyl alcohol, and slightly soluble in ether.

When it is topically applied to skin in gel form, 30% of active substance is easily absorbed.

Lidocaine is a local anesthetic substance. There are ointment, gel, spray or oral solution preparations to be used for local anesthetic effect and injectable preparations for use as local anesthetic. Lidocaine was first described in the patent document numbered US2441498. Chemical name is 2-(diethylamino)-N-(2,6-dimethylphenyl)acetamide and its chemical structure is as shown in formula II.

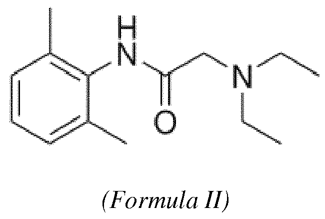

While hydrochloride salt of lidocaine is soluble in water, free base form is practically insoluble in water. It is soluble in alcohol and chloroform and freely soluble in benzene and ether.

KR20070059758 relates to transdermal application of non-steroidal antiinflammatory drugs (ibuprofen, ketoprofen, flurbiprofen, fenoprofen, naproxen, piroxicam, tenoxicam, isoxicam, meloxicam, indomethacin, aceclofenac or diclofenac) and local anesthetics (procaine, chlorcaine, tetracaine, prilocaine, lidocaine, mepivacaine, bupivacaine, ropivacaine or etidocaine). However, many active substances were mentioned as non-steroidal anti-inflammatory drugs and local anesthetics in this patent document, the combination of naproxen and lidocaine was not mentioned specifically.

Do 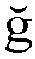 an E et al. studied anesthetic effect of intrauterine lidocaine and oral naproxen sodium combination in endometrial biopsy (Obstet Gynecol. 2004 February; 103(2):347-51). In this study, naproxen was given by oral route and local administration was not mentioned.

Gel products comprising mentioned active substances or combinations thereof exist in the market. For example, Naprosyn® gel, Naprodev® gel, Naponal® gel and Inaprol® gel are gel formulations comprising naproxen active substance.

Xylocaine® gel is a gel formulation that comprises lidocaine hydrochloride as active substance.

The product named Analgen-NF® comprises lidocaine hydrochloride and naproxen. In addition, Ultra Bengue® named product comprises lidocaine hydrochloride, naproxen, menthol, and methyl salicylate.

The mentioned formulations comprise lidocaine in the form of hydrochloride salt.

Hydrochloric acid is a strong electrolyte; therefore, it is completely ionized to H⁺ and Cl⁻ in water. Chloride is an anion that plays a significant role in providing the balance of body fluids. In addition, it is an important component of digestive fluid. However, excess and/or unnecessary chloride intake may lead to deterioration of body electrolyte balance, serious renal and cardiovascular disorders. In addition, it may cause an increase in blood pressure. Nowadays, many people take chloride more than adequate due to use of prepared/processed food and table salt (sodium chloride). Herein, there is a need for naproxen and lidocaine combinations that will prevent additional chloride intake. The Inventors of the present invention prevent additional chloride intake by combining naproxen active substance with free base form of lidocaine, instead of lidocaine hydrochloride salt.

Nevertheless hydrochloride salt of lidocaine active substance is well soluble in water, the free base form is insoluble in water. Until now in the art, lidocaine hydrochloride salt was preferred in the gel formulations, comprising naproxen and lidocaine, and lidocaine free base form was avoided.

Like lidocaine free base form, naproxen active substance is also practically insoluble in water. Therefore, it is very difficult to formulate these two water insoluble active substances in a stable and transparent gel form.

In addition, because naproxen and lidocaine are stable at neutral pH and basic pH, respectively, to formulate both active substances in a single dosage form poses a risk with respect to stability.

Considering the above mentioned prior art, the objective of the invention is to provide a formulation that comprises naproxen and free base form of lidocaine in a stable and transparent gel form.

### Detailed Description of the Invention

The inventors of the present invention surprisingly succeeded to formulate naproxen and free base of lidocaine in a single dosage form as a stable and transparent gel.

In this respect, the invention is directed to gel form formulations that comprise naproxen and free base of lidocaine.

Transparency of the product was measured by spectrometer. When this equipment is used, percent transmittance of the sample at 450 nm can be measured against a reference ethanol sample having 100% transmittance. Percent transmittance value of the formulation that is the subject of current invention is more than 70%, preferably more than 80%. It keeps this transmittance value throughout shelf-life.

The inventors of the present invention uses a special pH regulating agent-solvent system in order to keep both substances, which are stable at different pH values, stable in the gel formulation. The inventors found that pH regulating agent-solvent system provided an optimum dissolution medium for both active substances and a pH value that was necessary for both active substances to remain stable in the formulation. In the mentioned pH regulating agent-solvent system, naproxen and lidocaine dissolve without precipitation and remain stable throughout shelf life.

The object of the present invention, pH regulating agent which is necessary to constitute the pH regulating agent-solvent system can be selected among triethanolamine (TEA), amine base tromethamine, tetrahydroxypropyl ethylenediamine, diethanolamine, aminomethyl propanol, and/or sodium or ammonium hydroxide at a concentration less than 20% w/w.

Another object of the invention, triethanolamine (TEA) is preferably used as pH regulating agent. Triethanolamine content of the formulation is in the range of 5-35% of the total weight of formulation, preferably in the range of 7-15%.

Another object of the invention, the solvent which is necessary to constitute the pH regulating agent-solvent system can be selected from water, ethanol, isopropyl alcohol, propylene glycol, polyethylene glycol, benzyl alcohol, diethylether and dimethoxyethane, and/or mixture thereof.

In yet another object of the invention, water, ethanol, propylene glycol, and/or mixture thereof is used as solvent.

Ethanol amount of the formulation is in the range of 5-60% of the total weight of formulation, preferably approximately in the range of 15-25%.

Propylene glycol amount of the formulation is in the range of 5-30% of the total weight of formulation.

Another object of the invention, naproxen active substance can be dissolved in TEA/ethanol mixture. In this case, in order to adjust pH that naproxen effectively dissolves and remains stable, naproxen/TEA/ethanol proportions should be in the range of 1.0/0.4/2.5 to 1.0/3.3/7.0.

In a further object, naproxen active substance can be dissolved in TEA/water mixture. In this case, in order to adjust pH that naproxen effectively dissolves and remains stable, naproxen/TEA/water proportions should be in the range of 1.0/0.6/1.3 to 1.0/3.5/7.0.

In the present formulation, naproxen active substance is preferably dissolved in TEA/water/ethanol mixture.

By using water and ethanol together as solvent in the formulation, triethanolamine amount in the formula was decreased by 50-60%, antiseptic property was gained to the finished product, a good dissolution medium was provided, and precipitations that might develop in the gel after addition of lidocaine in the further steps of the process were prevented.

In one of the trials done by inventors, 10 g naproxen, 15 g ethanol, and 7 g triethanolamine were mixed. The pH value of the mixture was measured as 7.4. After a while, it was seen that naproxen in the mixture precipitated as agglomerates. However, in the mixture of 10 g naproxen, 40 g ethanol, and 7 g triethanolamine, the pH value was measured as 7.36 and it was observed that naproxen did not precipitate.

During manufacturing, inventors observed that addition of naproxen active substance to the formulation by dissolving in TEA/water, TEA/ethanol, or TEA/water/ethanol mixture was more advantageous than addition as dry powder. When it is directly added to the formulation as dry powder, naproxen does not demonstrate a homogenous distribution in the gel mixture and forms white agglomerates. In addition, powder naproxen added in a semi-solid gel leads to difficulties in the afterwards of the process. However, when it is added to the formulation after dissolving in TEA/water, TEA/ethanol, or TEA/water/ethanol mixtures, it homogenously dissolves in the gel formulation, no agglomeration forms, and no difficulty in the afterwards of the process occurs.

In object of the invention, lidocaine free base is dissolved in propylene glycol. Propylene glycol provided an ideal dissolution medium for lidocaine free base and increased penetration of the gel formulation to skin. In addition, it minimized the sticky feeling resulting from the use of viscosity enhancing agent in the formulation.

The pH range that the mentioned active substances remain stable in the formulation is 6.5-8.5, preferably 7.0-7.5.

The formulation of the invention comprises naproxen at 5-20% of the finished product weight. In other words, naproxen amount in the finished product is in the range of 5-20% of total formulation.

The formulation that is subject of the invention comprises lidocaine at 1-10% of the finished product weight. In other words, lidocaine amount in the finished product is in the range of 1-10% of total formulation.

The formulation of the invention comprises gelling agent, viscosity enhancing agent, and antioxidant substance in addition to pH regulating agent-solvent system.

Gelling agent in the formulation of the invention can be selected from carbomer polymers, cellulosic polymers, copolymers that hydrophobic chain is polyoxypropylene and/or hydrophilic chain is polyoxyethylene. Gelling agent in the formulation is selected from carbomer polymers. Carbomer polymers can be selected from Carbopol® 934, 940, 941, 971, 974, 980, or 981.

Gelling agent amount used in the formulation of the present invention is in the range of 0.5-4.0% of the total formulation weight.

Viscosity enhancing agents in the formulation can be selected from xanthan gum, guar gum, guar acetate, guar phthalate, guar acetate phthalate, and/or sodium carboxymethyl guar.

Viscosity enhancing agent amount in the formulation is in the range of 0.01-1.0% of the total formulation weight.

Another object of the invention, xanthan gum is preferably used as viscosity enhancing agent.

Antioxidant substance in the formulation can be selected from sodium metabisulphite, potassium metabisulphite, butyl hydroxy toluene (BHT), alpha tocopherol, ascorbic acid, ascorbyl palmitate, sodium sulphite, and/or sodium thiosulphate.

The formulation of the present invention presents many favorable features together such as not to give oily or stickiness sensation, spreading and absorption easily, not to leave stain, to remain stable throughout shelf life, and transparent appearance. In addition, the gel formulation does not comprise harmful preservatives such as paraben that may lead to skin irritation and contact dermatitis.

Unless otherwise specified, all weights and proportions mentioned in this description are in weight unit and all weight percentages are given comparing to the weight of total composition.

### Example 1

| **Content** | **Amount** |
|---|---|
| Naproxen | 5.00 g |
| Lidocaine free base | 2.50 g |
| Sodium sulphite | 0.06 g |
| Ethanol | 7.50 g |
| Carbopol | 0.50 g |
| Triethanolamine | 5.00 g |
| Guar gum | 0.05 g |
| Propylene glycol | 5.50 g |
| Deionized water | 23.89 g |
| **Total** | **50.00 g** |

Sodium sulphite, Carbopol and guar gum are mixed with water and ethanol in a mixing vessel. 1/2 of TEA is added to the mixture. In a second mixing vessel, lidocaine free base and propylene glycol are mixed. In a third mixing vessel, water and ethanol are mixed with naproxen and 1/2 of TEA. Contents of the second and the third mixing vessels are added to the first mixing vessel and it is mixed until a transparent gel is obtained. pH of the mixture is measured.

### Example 2

| **Content** | **Amount** |
|---|---|
| Naproxen | 5.00 g |
| Lidocaine free base | 2.50 g |
| Sodium metabisulphite | 0.10 g |
| Ethanol | 32.85 g |
| Carbopol | 1.00 g |
| Triethanolamine | 3.50 g |
| Xanthan gum | 0.05 g |
| Propylene glycol | 5.00 g |
| **Total** | **50.00 g** |

Sodium metabisulphite, Carbopol and xanthan gum are mixed with ethanol in a mixing vessel. 1/3 of TEA is added to the mixture. Lidocaine free base and propylene glycol are mixed in a second mixing vessel. Ethanol is mixed with naproxen and 2/3 of TEA in a third mixing vessel. Contents of the second and the third mixing vessels are added to the first mixing vessel and it is mixed until a transparent gel is obtained. pH of the mixture is measured.

### Example 3

| **Content** | **Amount** |
|---|---|
| Naproxen | 5.00 g |
| Lidocaine free base | 2.50 g |
| Butyl hydroxy toluene | 0.05 g |
| Carbopol | 0.50 g |
| Triethanolamine | 5.00 g |
| Guar gum | 0.05 g |
| Propylene glycol | 5.50 g |
| Deionized water | 31.40 g |
| **Total** | **50.00 g** |

Butyl hydroxy toluene, Carbopol and guar gum are mixed with water in a mixing vessel. 1/3 of TEA is added to the mixture. Lidocaine free base and propylene glycol are mixed in a second mixing vessel. Water, naproxen, and 2/3 of TEA are mixed in a third mixing vessel. Contents of the second and the third mixing vessels are added to the first mixing vessel and it is mixed until a transparent gel is obtained. pH of the mixture is measured.

In the stability studies at 25°C / 60% RH (Relative Humidity), 30°C / 65% RH, and 40°C / 75% RH, inventors observed that the composition subjected to invention was stable. Tables 1 and 2 present the results of stability studies of the formulation described in Example 1.

**Table 1 - Naproxen/Lidocaine Gel Impurity Stability Study**

| | Initial | 1st month 25 °C / 60% RH | 1st month 30 °C / 65% RH | 1st month 40 °C / 75% RH | 2nd month 25 °C / 60% RH | 2nd month 30 °C / 65% RH | 2nd month 40 °C / 75% RH | 3rd month 25 °C / 60% RH | 3rd month 30 °C / 65% RH | 3rd month 40 °C / 75% RH |
|---|---|---|---|---|---|---|---|---|---|---|
| Lido Impurity A | < LOQ | < LOQ | < LOQ | TE | < LOQ | < LOQ | < LOQ | < LOQ | < LOQ | < LOQ |
| Lido Impurity B | < LOQ | < LOQ | < LOQ | < LOQ | < LOQ | < LOQ | ND | ND | ND | < LOQ |
| Max. Unknown Impurity | 0.038 | 0.040 | 0.045 | 0.085 | 0.038 | 0.049 | 0.071 | 0.041 | 0.051 | 0.092 |
| | *RRT_1.86* | *RRT_1.85* | *RRT_4.59* | *RRT_5.62* | *RRT_1.86* | *RRT_1.86* | *RRT_1.86* | *RRT_1.86* | *RRT_1.86* | *RRT_5.25* |
| Total Impurity | 0.038 | 0.040 | 0.045 | 0.121 | 0.038 | 0.049 | 0.125 | 0.041 | 0.074 | 0.165 |
| *LOQ: Limit of quantification* | | | | | | | | | | |
| *ND: Not detected* | | | | | | | | | | |
| *RRT: Relative retention time* | | | | | | | | | | |

**Table 2 - Naproxen/Lidocaine Gel Assay Stability Study**

| | Initial | 1st month 25 °C / 60% RH | 1st month 30 °C / 65% RH | 1st month 40 °C / 75% RH | 2nd month 25 °C / 60% RH | 2nd month 30 °C / 65% RH | 2nd month 40 °C / 75% RH | 3rd month 25 °C / 60% RH | 3rd month 30 °C / 65% RH | 3rd month 40 °C / 75% RH |
|---|---|---|---|---|---|---|---|---|---|---|
| Lidocaine% | 99.5 | 100.0 | 99.6 | 99.4 | 100.0 | 100.6 | 99.1 | 98.8 | 99.6 | 97.2 |
| Naproxen% | 101.6 | 100.8 | 100.8 | 101.6 | 101.8 | 101.9 | 101.4 | 100.5 | 101.2 | 99.4 |

## Claims

1. A formulation in gel form wherein the formulation comprising naproxen and free base of lidocaine.

2. According to claim 1, a gel formulation comprises a pH regulating agent-solvent system.

3. A gel formulation according to claim 2, wherein pH regulating agent can be selected among triethanolamine (TEA), amine base tromethamine, tetrahydroxypropyl ethylenediamine, diethanolamine, aminomethyl propanol, and/or sodium or ammonium hydroxide at a concentration less than 20% w/w.

4. A gel formulation according to claim 3, wherein pH regulating agent is triethanolamine (TEA).

5. A gel formulation according to claim 4, wherein triethanolamine content of the formulation is in the range of 5-35% of the total weight of formulation, preferably in the range of 7-15%.

6. A gel formulation according to claim 2, wherein solvent can be selected among water, ethanol, isopropyl alcohol, propylene glycol, polyethylene glycol, benzyl alcohol, diethylether, dimethoxyethan, and/or mixture thereof.

7. A gel formulation according to claim 6, wherein solvent can be selected from water, ethanol, propylene glycol, and/or mixture thereof.

8. According to claims 6 and 7, ethanol amount of the formulation is in the range of 5-60% of the total weight of formulation, preferably approximately in the range of 15-25%.

9. According to claims 6 and 7, propylene glycol amount of the formulation is in the range of 5-30% of the total weight of formulation.

10. A gel formulation according to claim 1, wherein pH of the said formulation is in the range of 6.5-8.5, preferably 7.0-7.5.

11. A gel formulation according to claim 1, wherein said formulation comprises naproxen at 5-20% of the finished product weight.

12. A gel formulation according to claim 1, wherein said formulation comprises lidocaine free base at 1-10% of the finished product weight.

13. A gel formulation according to claims 1 and 2, wherein said formulation comprises gelling agent, viscosity enhancing agent, and antioxidant substance.

14. A gel formulation according to claim 13, wherein gelling agent in the formulation can be selected from carbomer polymers, cellulosic polymers, copolymers that hydrophobic chain is polyoxypropylene and/or hydrophilic chain is polyoxyethylene.

15. A gel formulation according to claim 13, wherein viscosity enhancing agent in the formulation can be selected from xanthan gum, guar gum, guar acetate, guar phthalate, guar acetate phthalate, and/or sodium carboxymethyl guar.

16. A gel formulation according to claim 13, wherein antioxidant substance in the formulation can be selected from sodium metabisulphite, potassium metabisulphite, butyl hydroxy toluene (BHT), alpha tocopherol, ascorbic acid, ascorbyl palmitate, sodium sulphite, and/or sodium thiosulphate.

## Patentansprüche

1. Eine Zusammensetzung in Gel Form, worin die die Zusammensetzung Naproxen und freie Base von Lidocain umfasst.

2. Nach dem Anspruch 1,worin Gelformulierung ein pH-regulierendes Wirkstoff-Lösungsmittel system umfasst.

3. Eine Gelformulierung nach Anspruch 2, worin pH-Regulierungsmittel aus Triethanolamin (TEA), Tromethamin auf Aminbasis, Tetrahydroxypropylethylendiamin, Diethanolamin, Aminomethylpropanol und / oder Natrium oder Ammoniumhydroxid in einer Konzentration von weniger als 20 % w/w ausgewählt werden kann.

4. Eine Gelformulierung nach Anspruch 3, worin pH-Regulierungsmittel Triethanolamin (TEA) ist.

5. Eine Gelformulierung nach Anspruch 4, worin der Triethanolamin Gehalt der Formulierung im Bereich von 5-35% des Gesamtgewichts liegt, vorzugsweise von 7-5%.

6. Eine Gelformulierung nach Anspruch 2, worin Lösungsmittel aus Wasser, Ethanol, Isopropylalkohol, Propylenglykol, Polyethylenglykol, Benzylalkohol, Diethylether, Dimethoxyethan und / oder Mischungen davon ausgewählt werden kann.

7. Eine Gelformulierung nach Anspruch 6, worin Lösungsmittel aus Wasser, Ethanol, Propylenglykol und / oder Mischungen davon ausgewählt werden kann.

8. Nach den Ansprüchen 6 und 7, liegt die Ethanolmenge der Formulierung im Bereich von 5-60% des Gesamtgewichts der Formulierung, vorzugsweise ungefähr von 15-25%.

9. Nach den Ansprüchen 6 und 7, liegt die Propylenglykolmenge der Formulierung im Bereich von 5-30% des Gesamtgewichts.

10. Eine Gelformulierung nach Anspruch 1, worin pH der genannten Formulierung im Bereich von 6,5-8,5 ist, vorzugsweise 7,0-7,5.

11. Eine Gelformulierung nach Anspruch 1, worin der genannten Formulierung Naproxen in einer Menge von 5-20% des Endproduktgewichts umfasst.

12. Eine Gelformulierung nach Anspruch 1, worin der genannten Formulierung freie Base von Lidocain in einer Menge von 1-10% des Endproduktgewichts umfasst.

13. Eine Gelformulierung nach den Ansprüchen 1 und 2, worin der genannten Formulierung, das Geliermittel, Viskositätserhöhendemittel und Antioxidans-Substanzen umfasst.

14. Eine Gelformulierung nach Anspruch 13, worin Geliermittel der Formulierung aus Carbomerpolymeren, Cellulosepolymeren, Copolymeren bei denen die hydrophobe Kette Polyoxypropylen und / oder die hydrophile Kette ist, ist Polyoxyethylen, ausgewählt werden kann.

15. Eine Gelformulierung nach Anspruch 13, worin Viskositätserhöhendesmittel der Formulierung aus Xanthangummi, Guargummi, Guaracetat, Guarphthalat, Guaracetatphthalat und / oder Natriumcarboxymethylguar ausgewählt werden kann.

16. Eine Gelformulierung nach Anspruch 13, worin Antioxidans-Substanzen der Formulierung aus Natriummetabisulfit, Kaliummetabisulfit, Butylhydroxytoluol (BHT), Alpha-Tocopherol, Ascorbinsäure, Ascorbylpalmitat, Natriumsulfit und / oder Natriumthiosulfat bestehen ausgewählt werden kann.

## Revendications

1. Formulation sous forme de gel dans laquelle la formulation comprend du naproxène et une base libre de lidocaine.

2. Selon la revendication 1, formulation de gel comprend un système d'agent de régulation du pH et de solvant.

3. Formulation de gel selon la revendication 2, dans laquelle l'agent de régulation du pH peut être choisi parmi la triéthanolamine (TEA), le trométhamine à base d'amine, la tétrahydroxypropyléthylènediamine, la diéthanolamine, l'aminométhyl propanol et / ou l'hydroxyde de sodium ou d'ammonium à une concentration inférieure à 20% poids/poids.

4. Formulation de gel selon la revendication 3, dans laquelle l'agent de régulation du pH est la triéthanolamine (TEA).

5. Formulation de gel selon la revendication 4, dans laquelle la teneur en triéthanolamine de la formulation est comprise entre 5% et 35% du poids total de la formulation, de préférence entre 7% et 15%.

6. Formulation de gel selon la revendication 2, dans laquelle le solvant peut être choisi parmi l'eau, l'éthanol, l'alcool isopropylique, le propylène glycol, le polyéthylène glycol, l'alcool benzylique, l'éther diéthylique, le diméthoxyéthane et / ou leur mélange.

7. Formulation de gel selon la revendication 6, dans laquelle le solvant peut être choisi parmi l'eau, l'éthanol, le propylène glycol et / ou leur mélange.

8. Selon les revendications 6 et 7, la quantité d'éthanol de la formulation est comprise entre 5% et 60% du poids total de la formulation, de préférence approximativement entre 15% et 25%.

9. Selon les revendications 6 et 7, la quantité de propylène glycol de la formulation est comprise entre 5% et 30% du poids total de la formulation.

10. Formulation de gel selon la revendication 1, dans laquelle le pH de ladite formulation est compris entre 6.5 et 8.5, de préférence entre 7.0 et 7.5.

11. Formulation de gel selon la revendication 1, dans laquelle ladite formulation comprend du naproxène à 5-20% du poids du produit fini.

12. Formulation de gel selon la revendication 1, dans laquelle ladite formulation comprend une base libre de lidocaïne à 1-10% du poids du produit fini.

13. Formulation de gel selon les revendications 1 et 2, dans laquelle ladite formulation comprend un agent gélifiant, un agent améliorant la viscosité et une substance antioxydante.

14. Formulation de gel selon la revendication 13, dans laquelle l'agent gélifiant dans la formulation peut être choisi parmi les polymères de carbomère, les polymères cellulosiques, les copolymères dont la chaîne hydrophobe est le polyoxypropylène et / ou la chaîne hydrophile est le polyoxyéthylène.

15. Formulation de gel selon la revendication 13, dans laquelle l'agent améliorant la viscosité dans la formulation peut être choisi parmi la gomme de xanthane, la gomme de guar, l'acétate de guar, le phtalate de guar, l'acétate phtalate de guar et / ou le carboxyméthylguar sodique.

16. Formulation de gel selon la revendication 13, dans laquelle la substance antioxydante dans la formulation peut être choisie parmi le métabisulfite de sodium, le métabisulfite de potassium, le butylhydroxytoluène (BHT), l'alpha tocophérol, l'acide ascorbique, le palmitate d'ascorbyle, le sulfite de sodium et / ou le thiosulfate de sodium.
